(19) Europäisches Patentamt / European Patent Office / Office européen des brevets

(11) **EP 2 626 092 B1**

(12) **EUROPEAN PATENT SPECIFICATION**

(45) Date of publication and mention
of the grant of the patent:
**05.08.2015 Bulletin 2015/32**

(51) Int Cl.:
*A61L 31/14* (2006.01)　　*A61L 31/04* (2006.01)
*A61L 27/20* (2006.01)　　*A61L 24/00* (2006.01)
*A61K 47/36* (2006.01)　　*A61K 47/40* (2006.01)
*A61L 24/08* (2006.01)

(21) Application number: **12765149.5**

(22) Date of filing: **12.03.2012**

(86) International application number:
**PCT/CN2012/072170**

(87) International publication number:
**WO 2012/130031 (04.10.2012 Gazette 2012/40)**

(54) **MEDICAL ABSORBABLE HEMOSTATIC MATERIAL FOR BONE WOUNDS AND PREPARATION METHOD THEREFOR**

RESORBIERBARES MEDIZINISCHES BLUTSTILLUNGSMATERIAL FÜR KNOCHENWUNDEN UND HERSTELLUNGSVERFAHREN DAFÜR

MATÉRIAU MÉDICAL HÉMOSTATIQUE ABSORBABLE POUR LES LÉSIONS OSSEUSES ET PROCÉDÉ DE PRÉPARATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.04.2011 CN 201110081233**

(43) Date of publication of application:
**14.08.2013 Bulletin 2013/33**

(73) Proprietor: **Zhuhai Ortus Biotechnology Co., Ltd.
Guangdong (CN)**

(72) Inventors:
• **YANG, Peng**
  **Guangdong 510095 (CN)**
• **XING, Fei**
  **Guangdong 510095 (CN)**
• **SONG, Xiangrui**
  **Guangdong 510095 (CN)**
• **SHEN, Nan**
  **Guangdong 510095 (CN)**

(74) Representative: **Hannke, Christian
Hannke Bittner & Partner
Patent- und Rechtsanwälte
Prüfeninger Strasse 1
93049 Regensburg (DE)**

(56) References cited:
WO-A1-2005/034816　　CN-A- 1 872 351
CN-A- 101 361 986　　CN-A- 102 139 123
GB-A- 2 463 523　　US-A- 4 439 420
US-A1- 2005 065 214　　US-A1- 2010 298 264

**Description**

FIELD OF THE INVETION

The present invention belongs to the field of medical technology, and particularly relates to an absorbable hemostatic and wound healing promoting material for bone wounds, and a preparation method thereof.

BACKGROUND OF THE INVENTION

Medical sterile bone wax is an essential auxiliary material for hemostasis of cancellous bone during orthopedic surgeries, thoracic surgeries and neurological surgeries, by virtue of its tamponade action based on its unique hardness, toughness and viscosity. At present, many types of bone wax have been self-made by hospitals without complying with any uniform formula or standard, and these bone waxes are mainly made from beeswax and vaseline; beeswax, refined sesame oil and salicylic acid; and beeswax, peanut oil and aspirin, and the like. Most of these bone waxes do not have required hardness, toughness or viscosity, and always have poor hemostasis effects. Main bone wax products commercially available in China at present include a medical bone wax provided by Johnson & Johnson, America, and a medical sterile bone wax (Chinese Patent No. 00110393.8) developed by General Hospital of Shenyang Military Region, China, with the latter approved by SFDA in 2003. Though the self-made bone wax, the bone wax produced by Johnson & Johnson and the bone wax developed by the General Hospital of Shenyang Military Region all can stop bleeding, these types of bone wax are non-degradable and remain in the body as a permanent foreign matter, and in the aspect of non-degradability, there is no substantial difference between these types of bone wax and conventional bone waxes, therefore nonunions, bacterial infections, rejections and other complications could be elicited by these non-degradable materials.

Chinese Patent No. 200610093091.3 discloses a water-soluble bone wax and a preparation method thereof, comprising a copolymer obtained by the co-polymerization of polyoxyethylene and polyoxypropylene, and a sorbitan fatty acid ester as an emulsifier, characterized in that the dissolution and elimination of the copolymer of polyoxyethylene and polyoxypropylene in the body is enhanced by improving the solubility of the bone wax. However, foreign body responses still exist in the body when using the bone wax, and the speed of degradation is very slow, leading to a long periods of time for the degradation.

Chinese Patent No. 200510035251.4 discloses a medical hemostatic material which can be substituted for the bone wax, its preparation method comprises melt-blending a base material and an adjuvant, wherein the base material is prepared by a melt-polycondensation of a copolymer/mixture of low molecular weight poly-DL-lactic acids or poly-L-lactic acids or polyglycolic acids, the adjuvant is consisted of a polyether diol, a polyether triol, a high molecular weight polylactic acid, a high molecular weight copolymer of glycolide and lactide, a high molecular weight copolymer of caprolactone and lactide or a high molecular weight copolymer of caprolactone and glycolide. The hemostatic material uses high molecular polymers as raw materials that can be degraded and absorbed by human bodies, however, a long-term degradation period is needed.

German Patent No. 3229540.5 discloses a method for preparing absorbable bone waxes, and the bone waxes consist of polyester-oligomers of hydroxycarboxylic acids, and the waxes have average molecular weights in the range from about 200-1500. According to the patent, monofunctional and/or difunctional hydroxide groups or carboxyl groups or carboxylic anhydrides and/or amine compounds may be used to regulate the average molecular weight of the polyester oligomers. The resulting bone wax has poor performances in flexibility and scalability, besides, unreacted monomers in the wax may remain in the body, which may elicit a relatively intense stimulation of human tissues.

American Patent No. 5143730 discloses a method for preparing an absorbable bone wax, in this method, a low molecular weight polyglycolic acid or a low molecular weight polylactic acid is reacted with equimolar quantities of calcium carbonate at a high temperature to produce calcium polyglycolate or calcium polylactate, then hydroxyl apatite is added to the above reaction system to obtain the resulting bone wax, the bone wax made by this method has a long-term degradation period in the body, in addition, though the hydroxyl apatite has exhibited a good biocompatibility in the body, it is a non-degradable material.

US 4439420 A discloses an absorbable hemostatic composition for use in the control of osseous hemorrhage, comprising a mixture of calcium stearate and dextran and a component comprising a biocompatible base consisting of a natural or synthetic oil or wax which is soluble in the body.

WO 2005/034816 A1 relates to an hemostatic composition which comprises a mixture of a polysaccharide such as dextran, a polyol and a vegetable oil.

US 2005/06214 A1 discloses an absorbable bone hemostatic agent comprising a polysaccharide such as dextran, a polyol such as glycerol, a vegetable oil and an emulsifying agent such as Tween.

An absorbable biomaterial hemostatic material AristaTm AH@ (Absorbable Surgical Hemostat, Medafor Inc., USA) has been studied in clinical research for its potential use as a substitution for bone wax. Because the product is derived from plant polysaccharides, its degradation in the body is rapid and will not elicit immune reaction or foreign body reaction, so it is a highly safe hemostat in clinical use, and becomes a beloved hemostat for its excellent hemostasis effects. However, AristaTm AH@ is in the form of powder, when performing hemostasis on injured cancellous bones, the hemostasis might become very difficult if the bone is vertical or fornix-shaped, because quite a few powder may be peeled off under the action of gravity.

SUMMARY OF THE INVENTION

In order to solve the problem with existing bone wax that cannot be degraded in human body or animal body and thus affecting the healing, it is therefore an object of the invention to provide a hemostatic and wound healing promoting material for bone wounds, the material of the present invention is completely degradable in the bodies and can promote the healing of bone tissues, additionally, the material can be a substitution for bone wax, thus doctors' operating habits will not be changed.

Another object of the present invention is to provide a method of preparing a medical absorbable hemostatic and wound healing promoting material for bone wounds.

The present invention provides the following technical solutions to achieve the above objects.

A medical absorbable hemostatic material for bone wounds, consisting of 40-95% of a base material and 5-60% of an adjuvant, based on weight percent; wherein the base material is an oligosaccharide, an polysaccharide or a mixture of the oligosaccharide and the polysaccharide; and the adjuvant includes: (1) one or more polyhydric alcohols, and (2) one or more vegetable oils, and (3) one or more emulsifying agents.

Preferably, the base material of the present invention is a mixture of the oligosaccharide and the polysaccharide, or a polysaccharide;

Preferably, the adjuvant includes (1) one polyhydric alcohol, and

(2) one vegetable oil, and
(3) one emulsifying agent.

The mixture of the oligosaccharide and the polysaccharide contains 10-90% of the oligosaccharide and 10-90% of the polysaccharide, based on weight percent.

The oligosaccharide is selected from the group consisting of maltose, isomaltose, coupling sugar, soybean sugar, galactose, fructose, sucrose, xylose, maltitol prepared by hydrogenation reactions, soybean sugar alcohol prepared by hydrogenation reactions, galactitol prepared by hydrogenation reactions, fructose alcohol prepared by hydrogenation reactions, sucrose alcohol prepared by hydrogenation reactions and xylitol prepared by hydrogenation reactions; the oligosaccharide is preferably selected from the group consisting of maltose, coupling sugars, soybean sugar, galactose, fructose, sucrose and xylose; the oligosaccharide has a molecular weight in the range from 160 to 20,000, and the oligosaccharide has a viscous power in the range from 50 to 700 g.mm at room temperature.

The polysaccharide is selected from the group consisting of carboxymethyl cellulose, carboxymethyl starch, carboxypropyl methylcellulose, hydroxypropyl starch, pre-gelatinized starch, crosslinked carboxymethyl cellulose, pharmaceutically acceptable starch, dextrin and dextrin derivative; the dextrin derivative comprises: alpha-cyclodextrin, beta-cyclodextrin, hydroxypropyl beta-cyclodextrin, carboxymethyl beta-cyclodextrin, sulfobutyl ether-$\beta$-cyclodextrin, gamma-cyclodextrin; the polysaccharide is preferably selected from the group consisting of carboxymethyl cellulose, carboxymethyl starch, carboxypropyl methylcellulose, hydroxypropyl starch, pre-gelatinized starch, pharmaceutically acceptable starch, dextrin; the polysaccharide has a molecular weight in the range from 10,000 to 1,000,000, and has a water absorption rate above 5, and the polysaccharide has a viscous power in the range from 10 to 300 g.mm at room temperature.

The adjuvant includes 30-70% of the polyhydric alcohol, 20-60% of the vegetable oil, and 1-10% of the emulsifying agent, based on weight percent; the polyhydric alcohol comprises at least one selected from medical glycerin and propylene glycol; the vegetable oil comprises at least one selected from the group consisting of olive oil, soybean oil, hydrogenated soybean oil, hydrogenated castor oil and refined corn oil; and the emulsifying agent comprises at least one selected from the group consisting of soybean phospholipid, Tween, polysorbate and sucrose stearate.

The absorbable hemostatic material for bone wounds has a bond strength $\geq$0.08 mPa, has a bond strength under water immersion $\geq$0.03 mPa, and has an amount of water absorption $\geq$0.5 ml/g.

Preferably, the absorbable hemostatic material for bone wounds has a bond strength $\geq$0.16 mPa, has a bond strength under water immersion $\geq$0.06 mPa, and has an amount of water absorption $\geq$2.0 ml/g.

Most preferably, the absorbable hemostatic material for bone wounds has a bond strength $\geq$0.19 mPa, has a bond strength under water immersion $\geq$0.16 mPa, and has an amount of water absorption $\geq$6.0 ml/g.

The adjuvant of the present invention is consisted of the polyhydric alcohol, the vegetable oil and the emulsifying agent, wherein the polyhydric alcohol is easily soluble in water, while the vegetable oil is insoluble in water, the hemostatic material thus exhibits water resistance and eventually dissolves, so the hemostatic material's water-resistance time can be adjusted according to the ratio of polyhydric alcohol and vegetable oil in the material. Since the mixture of polyhydric alcohol and vegetable oil has a poor stability, a certain amount of emulsifying agent can be added to the mixture followed by a blending to produce a chemical bonding between the polyhydric alcohol and the vegetable oil, in this way, the stability of the hemostatic material is greatly enhanced.

A method for preparing a medical absorbable hemostatic material for bone wounds comprises: blending a base material and an adjuvant at prescribed amounts through chemical blending and latex blending, cooling to form a solid lump, packaging, and sterilizing; wherein the blendings of the base material and the adjuvant are conducted at a temperature between 40 °C and 80 °C.

In the above method, the chemical blending and the latex blending comprise the steps of:

(6) pretreatment of the base material: washing a polysaccharide with a solvent followed by a drying process to remove impurities and microorganisms from the polysaccharide, and dissolving an oligosaccharide followed by a filtering process to remove impurities and microorganisms from the oligosaccharide;

(7) putting a prescribed amount of the adjuvant into a reaction kettle, then sealing and vacuumizing the reaction kettle, heating the adjuvant to a temperature between 30 °C and 50 °C and maintaining the temperature, stirring the adjuvant for 0.5-2 hours.

(8) putting a prescribed amount of the base material into the reaction kettle, then sealing and vacuumizing the reaction kettle, heating the mixture in the reaction kettle to a temperature between 40°C and 80°C and maintaining the temperature, stirring the mixture for 2-6 hours.

(9) pouring the mixture in the reaction kettle into a mold for molding, immediately placing the mold in an environment having a temperature between 0°C and 4°C, cooling the mold for 0.5-2 hours to obtain a solid lump product;

(10) packaging the product followed by sterilization through irradiation or oxirane.

In the above method, the vacuum degree in the reaction kettle is between 10KPa and 30KPa after the vacuum processes. The purpose of the vacuum process is to remove the bubbles in the mixed materials generated during the blending process, such that the mixed materials can be more homogeneous, which may help improve various performances of the hemostatic material.

The medical absorbable hemostatic material for bone wounds of the present invention can be used for hemostasis of bone wounds in humans and animals, and can also be used for promoting healing of bone wounds in humans and animals.

The hemostatic mechanism of the absorbable hemostatic material for bone wounds of the present invention is considered to be as follows: the material is effective in stopping bleeding by virtue of its tamponade action based on its unique bond strength, besides, the material can absorb water from the blood. Consequently, once the hemostatic material is in contact with the blood, the blood can be concentrated, resulting in an aggregation of blood platelets, red blood cells and the clotting proteins (such as thrombins and fibrinogens), thereby the process of natural blood coagulation is accelerated, and the rapid hemostasis also has favorable effects.

The base material as well as the adjuvant in the hemostatic material of the present invention can be easily degraded in the human bodies or animal bodies, and the resulting degradation products is then absorbed and metabolized by the bodies without residuals or toxic side effects, the absorption of the hemostatic material usually takes a few weeks, depending on the dose and the applying site.

Comparing with the prior art, the advantages of the medical absorbable hemostatic material for bone wounds of the present invention are as follows:

The medical absorbable hemostatic material for bone wounds of the present invention is in the form of solid lump which facilities its use, it has favorable effects in stopping bleeding by virtue of its ideal hardness, toughness and viscosity, it can also concentrate the blood and aggregate blood platelets, red blood cells and the clotting proteins, thereby the process of natural blood coagulation is accelerated which results in a rapid hemostasis; the material is biodegradable in human bodies and the degradation products has no toxic side effects on the bodies, which avoids nonunions, bacterial infections, rejections and other complications that might be elicited when applying conventional hemostatic drug on injured bones, thereby the material will not stimulate human tissues and is highly safe for use. In addition, both the base material and the adjuvant are widely used pharmaceutical excipients around the world, they have high safeties, well biocompatibilities, low costs and easy preparing processes, thereby the hemostatic material of the present invention has reliable safety and has clinically promotional value.

DETAILED DESCRIPTION OF THE PREFERRED EMBODIMENTS OF THE INVENTION

The details of the present invention will be more fully understood and appreciated from the following description, taken in conjunction with the embodiments. While the preferred embodiments of the invention will be described below, these embodiments are not limitations on the protection scope of the present invention,

## EXAMPLE 1

Carboxymethyl cellulose was wash with alcohol followed by a drying process to obtain powders, maltose was dissolved in distilled water followed by a filtering process and the filtrate was concentrated to obtain a maltose syrup containing ≥75% by weight of solute maltose.

23g of medical glycerol, 3 g of Tween 80 and 10g of olive oil were put into a reaction kettle, then the reaction kettle was sealed and vacuumized to obtain a vacuum degree of 10KPa, the mixture in the reaction kettle was heated to a temperature of 50°C, the temperature was maintained and the mixture was stirred for 30min; a maltose syrup containing 38g of the carboxymethyl cellulose and 26g of the maltose was put into the reaction kettle, then the reaction kettle was sealed and vacuumized, the mixture in the reaction kettle was heated to a temperature of 80°C and a vacuum degree of 10KPa was kept, the mixture was then stirred continuously at a slow speed for 2 hours. The pasty fluid in the reaction kettle was poured into a Teflon mold and the mold was immediately placed in a freezer having a temperature of 4°C, the mold was cooled for 120 min. Thereby a solid lump product (Sample A) being optionally shapable and having a certain mechanical strength was produced. Finally, the product was sealed and packaged followed by a sterilization process through irradiation.

## EXAMPLE 2

Hydroxypropyl starch was wash with alcohol followed by a drying process to obtain powders, sucrose was dissolved in distilled water followed by a filtering process to remove impurities and the filtrate was dried to powders. 12g of glycerol, 0.5g of polysorbate and 7.5g of refined corn oil were put into a reaction kettle, then the reaction kettle was sealed and vacuumized to obtain a vacuum degree of 20KPa, the mixture in the reaction kettle was heated to a temperature of 30 °C, the temperature was maintained and the mixture was stirred for 120min; 63g of the hydroxypropyl starch and 17g of the sucrose were put into the reaction kettle, then the reaction kettle was sealed and vacuumized, the mixture in the reaction kettle was heated to a temperature of 40 °C and a vacuum degree of 20KPa was kept, the mixture was then stirred continuously at a slow speed for 6 hours. The pasty fluid in the reaction kettle was poured into a Teflon mold and the mold was immediately placed in a freezer having a temperature of 2 °C, the mold was cooled for 90 min. Thereby a solid lump product (Sample B) being optionally shapable and having a certain mechanical strength was produced. Finally, the product was sealed and packaged followed by a sterilization process through oxirane.

## EXAMPLE 3

Carboxymethyl starch was wash with alcohol followed by a drying process to obtain powders. 31 g of glycerol, 2g of Tween 80 and 9g of soybean oil were put into a reaction kettle, then the reaction kettle was sealed and vacuumized to obtain a vacuum degree of 30KPa, the mixture in the reaction kettle was heated to a temperature of 30 °C, the temperature was maintained and the mixture was stirred for 60min; 58g of the carboxymethyl starch was put into the reaction kettle, then the reaction kettle was sealed and vacuumized, the mixture in the reaction kettle was heated to a temperature of 50 °C and a vacuum degree of 30KPa was kept, the mixture was then stirred continuously at a slow speed for 4.5 hours. The pasty fluid in the reaction kettle was poured into a Teflon mold and the mold was immediately placed in a freezer having a temperature of 0 °C, the mold was cooled for 30 min. Thereby a solid lump product (Sample C) being optionally shapable and having a certain mechanical strength was produced. Finally, the product was sealed and packaged followed by a sterilization process through oxirane.

## EXAMPLE 4

Fructose was dissolved in distilled water followed by a filtering process to remove impurities, and the filtrate was dried to powders. 15g of glycerol, 1g of polysorbate and 7g of hydrogenated castor oil were put into a reaction kettle, then the reaction kettle was sealed and vacuumized to obtain a vacuum degree of 30KPa, the mixture in the reaction kettle was heated to a temperature of 50 °C, the temperature was maintained and the mixture was stirred for 30min; 77g of the fructose was put into the reaction kettle, then the reaction kettle was sealed and vacuumized, the mixture in the reaction kettle was cooled to a temperature of 40 °C and a vacuum degree of 30KPa was kept, the mixture was then stirred continuously at a slow speed for 1.5 hours. The pasty fluid in the reaction kettle was poured into a Teflon mold and the mold was immediately placed in a freezer having a temperature of 4°C, the mold was cooled for 120 min. Thereby a solid lump product (Sample D) being optionally shapable and having a certain mechanical strength was produced. Finally, the product was sealed and packaged followed by a sterilization process through irradiation.

**EXAMPLE 5**

Pre-gelatinized starch was wash with alcohol followed by a drying process to obtain powders, maltose was dissolved in distilled water followed by a filtering process and the filtrate was concentrated to obtain a maltose syrup containing ≥75% by weight of solute maltose. 16g of glycerol, 1g of Tween 80 and 10g of refined corn oil were put into a reaction kettle, then the reaction kettle was sealed and vacuumized to obtain a vacuum degree of 10KPa, the mixture in the reaction kettle was heated to a temperature of 30 °C, the temperature was maintained and the mixture was stirred for 30min; 63g of the pre-gelatinized starch and the maltose syrup containing 10g of the maltose were put into the reaction kettle, then the reaction kettle was sealed and vacuumized, the mixture in the reaction kettle was heated to a temperature of 50 °C and a vacuum degree of 10KPa was kept, the mixture was then stirred continuously at a slow speed for 3.5 hours. The pasty fluid in the reaction kettle was poured into a Teflon mold and the mold was immediately placed in a freezer having a temperature of 4°C, the mold was cooled for 120 min. Thereby a solid lump product (Sample E) being optionally shapable and having a certain mechanical strength was produced. Finally, the product was sealed and packaged followed by a sterilization process through oxirane.

**EXAMPLE 6**

Dextrin was wash with alcohol followed by a drying process to obtain powders, sucrose was dissolved in distilled water followed by a filtering process to remove impurities, and the filtrate was dried to powders. 13g of glycerol, 1g of polysorbate and 10g of refined corn oil were put into a reaction kettle, then the reaction kettle was sealed and vacuumized to obtain a vacuum degree of 30KPa, the mixture in the reaction kettle was heated to a temperature of 30 °C, the temperature was maintained and the mixture was stirred for 90min; 60g of the dextrin and 16g of the sucrose were put into the reaction kettle, then the reaction kettle was sealed and vacuumized, the mixture in the reaction kettle was heated to a temperature of 50 °C and a vacuum degree of 30KPa was kept, the mixture was then stirred continuously at a slow speed for 4 hours. The pasty fluid in the reaction kettle was poured into a Teflon mold and the mold was immediately placed in a freezer having a temperature of 4°C, the mold was cooled for 60 min. Thereby a solid lump product (Sample F) being optionally shapable and having a certain mechanical strength was produced. Finally, the product was sealed and packaged followed by a sterilization process through irradiation.

**EXAMPLE 7**

Pharmaceutically acceptable starch was wash with alcohol followed by a drying process to obtain powders. 20g of glycerol, 10g of propylene glycol, 0.3g of polysorbate, 0.3g of sucrose stearate and 29.4g of refined corn oil were put into a reaction kettle, then the reaction kettle was sealed and vacuumized to obtain a vacuum degree of 30KPa, the mixture in the reaction kettle was heated to a temperature of 30 °C, the temperature was maintained and the mixture was stirred for 90min; 40g of the pharmaceutically acceptable starch was put into the reaction kettle, then the reaction kettle was sealed and vacuumized, the mixture in the reaction kettle was heated to a temperature of 50 °C and a vacuum degree of 30KPa was kept, the mixture was then stirred continuously at a slow speed for 4 hours. The pasty fluid in the reaction kettle was poured into a Teflon mold and the mold was immediately placed in a freezer having a temperature of 4°C, the mold was cooled for 60 min. Thereby a solid lump product (Sample G) being optionally shapable and having a certain mechanical strength was produced. Finally, the product was sealed and packaged followed by a sterilization process through irradiation.

**EXAMPLE 8**

Hydroxypropyl starch was wash with alcohol followed by a drying process to obtain powders, sucrose was dissolved in distilled water followed by a filtering process to remove impurities, and the filtrate was dried to powders. 21 g of glycerol, 3g of polysorbate, 3g of refined corn oil and 3g of olive oil were put into a reaction kettle, then the reaction kettle was sealed and vacuumized to obtain a vacuum degree of 30KPa, the mixture in the reaction kettle was heated to a temperature of 30 °C, the temperature was maintained and the mixture was stirred for 90min; 52g of the hydroxypropyl starch and 18g of the sucrose were put into the reaction kettle, then the reaction kettle was sealed and vacuumized, the mixture in the reaction kettle was heated to a temperature of 50 °C and a vacuum degree of 30KPa was kept, the mixture was then stirred continuously at a slow speed for 4 hours. The pasty fluid in the reaction kettle was poured into a Teflon mold and the mold was immediately placed in a freezer having a temperature of 4°C, the mold was cooled for 60 min. Thereby a solid lump product (Sample H) being optionally shapable and having a certain mechanical strength was produced. Finally, the product was sealed and packaged followed by a sterilization process through irradiation.

## EXAMPLE 9

Carboxymethyl cellulose was wash with alcohol followed by a drying process to obtain powders, maltose was dissolved in distilled water followed by a filtering process and the filtrate was concentrated to obtain a maltose syrup containing ≥75% by weight of solute maltose.

1.5g of medical glycerol, 0.5g of Tween 80 and 3g of olive oil were put into a reaction kettle, then the reaction kettle was sealed and vacuumized to obtain a vacuum degree of 20KPa, the mixture in the reaction kettle was heated to a temperature of 40 °C, the temperature was maintained and the mixture was stirred for 60min; a maltose syrup containing 45g of the carboxymethyl cellulose and 50g of the maltose was put into the reaction kettle, then the reaction kettle was sealed and vacuumized, the mixture in the reaction kettle was heated to a temperature of 60 °C and a vacuum degree of 20KPa was kept, the mixture was then stirred continuously at a slow speed for 5 hours. The pasty fluid in the reaction kettle was poured into a Teflon mold and the mold was immediately placed in a freezer having a temperature of 4°C, the mold was cooled for 120 min. Thereby a solid lump product (Sample I) being optionally shapable and having a certain mechanical strength was produced. Finally, the product was sealed and packaged followed by a sterilization process through irradiation.

In the above embodiments, the oligosaccharide has a molecular weight in the range from 160 to 20,000, and the oligosaccharide has a viscous power in the range from 50 to 700 g.mm at room temperature; the polysaccharide has a molecular weight in the range from 10,000 to 1,000,000, and has a water absorption rate above 5, and the polysaccharide has a viscous power in the range from 10 to 300 g.mm at room temperature.

## EXPERIMENT 1

Table 1. Physical parameters of different samples produced by different methods in the above examples

| Sample | Appearance | Amount of Water Absorption ml/g | Operational Feasibility | Drying Time h/ Crack Resistance | Dissolution Time (min) | Bond Strength mPa | Bond Strength Under Water Immersion mPa |
|---|---|---|---|---|---|---|---|
| Sample A | Excellent | 47 | Good | 72/ Excellent | 49 | 0.47 | 0.31 |
| Sample B | Excellent | 5 | Excellent | 33/ Excellent | 37 | 0.24 | 0.17 |
| Sample C | Good | 27 | Good | 20/ Good | 21 | 0.19 | 0.16 |
| Sample D | Good | 0.5 | Excellent | 16/ Good | 35 | 0.09 | 0.03 |
| Sample E | Excellent | 4 | Excellent | 10/ Good | 30 | 0.33 | 0.21 |
| Sample F | Good | 3.5 | Excellent | 36/ Excellent | 20 | 0.22 | 0.14 |
| Sample G | Excellent | 15 | Excellent | 21 / Good | 25 | 0.08 | 0.03 |
| Sample H | Excellent | 10 | Excellent | 30/ Excellent | 23 | 0.28 | 0.19 |
| Sample I | Good | 6 | Good | 34/ Excellent | 18 | 0.25 | 0.18 |
| Note: Appearance: evaluation on homogeneity, clotting and cracking of the material | | | | | | | |

Amount of Water Absorption ml/g: the maximum amount of water absorption before the disintegration of lump sample / weight of the lump sample.

Operational Feasibility: each sample was evenly applied over a sample plate by an experimenter to experience whether the application is easy or not so as to determine the convenience of use.

Drying Time: each sample was evenly applied over a sample plate by an experimenter to determine the time needed for the complete drying of the sample.

Dissolution Time (h): each sample was immersed into water at a temperature of 37 °C by an experimenter to determine the time needed before the dissolution of the sample.

Bond Strength: according to ASTM D952 Standard Test Method, each sample was coated onto a lower testing plate of two metal testing plate of Lloyd Instruments materials testing machines- LS1, and the coating thickness was 2mm, then an upper testing plate was pulled back to be fully contacted with the sample, then the test began, with a testing speed of 0.01-0.02mm/min; collected data was analyzed by NEXYGEN Plus data analysis software to obtain results.

Bond Strength Under Water Immersion: each sample was coated onto an upper testing plate of two metal testing plate of Lloyd Instruments materials testing machines- LS1, and the coating thickness was 2mm, after both the upper testing plate and the sample had been immersed into water for 50 seconds, the upper testing plate was pulled back, such that the sample was fully contacted with a lower testing plate, then the test began, the operation parameters and data analysis process were the same as above.

## EXPERIMENT 2

Experimental Purpose:

The experiment was conducted to examine the efficacy of the absorbable hemostatic and wound healing promoting material for bone wound of the present invention in hemostasis and healing of rabbit cranial bone.

Tested samples:

Sample A, Sample B, Sample C, Sample D, Sample E, Sample F and a commercially available bone wax.

Experimental Methods

24 healthy adult New Zealand rabbits (2.0-2.5kg) were provided, and 4 bone defects would be drilled for each of these rabbits, then 8 groups, i.e., Control Group, Sample A Group, Sample B Group, Sample C Group, Sample D Group, Sample E Group, Sample F Group and Bone Wax Group were arranged randomly, thus there were 12 bone defects in each group. The New Zealand rabbits were anesthetized by an intravenous injection of 3% sodium pentobarbital solution (1ml/kg), and the limbs of rabbits were fixed to an operating table at prone position. A sagittal scalp incision of approximately 4 cm was made, and the periosteum was meticulously stripped off to expose the parietal skull. A 6mm-diameter drill was used to drill two circular defects on both sides of the middle raphe of cranial bone, all defects penetrated through the layer of parietal skull (where the calvarial thickness was uniform) and were not cross the middle raphe. The defects were randomly covered with the absorbable hemostatic and wound healing promoting material for bone wounds of the present invention or the bone wax or left empty, as a control. Absorbable sutures were used to suture the periosteum and the scalp fur during the surgery, after a sterile bandaging operation, the rabbits were allowed to further recover in their cages. The rabbits were given a daily intramuscular injection of 40U gentamicin for continuous 3 days after the surgery to prevent infection. These animals were observed for general conditions at daily intervals.

The hemostasis efficacy of different experimental materials was observed during the surgery, the amount of specimens obtaining successful hemostasis and the corresponding successful hemostasis rate were recorded. 6 weeks after the surgery, the animals were sacrificed by air embolism, then residues of the experimental materials were examined at the sites applied with the hemostatic and wound healing promoting material for bone wounds of the present invention or the bone wax; specimens of defects of cranial bone were harvested with a rim of surrounding bone at least 1.5 cm from the defect edge and including the adjacent pericranium and dura. The cranial bone specimens were fixed in 70% ethanol. The bone healing score was determined by an experimenter: bone healing was evaluated in all defects using the group-described healing score below: healing score 0=No visible defect; 1=minimal visible defect; 2=moderate visible defect; 3=extensive visible defect.

## RESULTS

After the samples had been applied, the hemostatic process was observed by an experimenter for 1 min, it was concluded that the absorbable hemostatic and wound healing promoting material for bone wounds of the present invention, including Sample A, Sample B, Sample C, Sample D, Sample E and Sample F, and the bone wax all could effectively stop bleeding, however, one defect that had been applied with Sample D bled again, and this was recorded as a failure hemostasis. The bleeding of defects in Control Group could not be stopped by pressing for 1min. When comparing with the control, the tested hemostatic sealing material samples, including Sample A, Sample B, Sample C, Sample D, Sample E, Sample F and the bone wax, all significantly decreased the bleeding volume (P<001) and had short hemostatic time. See Table 2.

Table 2. Effects of different experimental materials on hemostasis of bleeding cranial bone in rabbits

| Group | The amount of specimens obtaining successful hemostasis /total amount of tested specimens | Successful hemostasis rate (%) |
|---|---|---|
| Control Group | 0/12 | 0 |
| Sample A Group | 12**/12 | 100 |
| Sample A Group | 12**/12 | 100 |
| Sample A Group | 12**/12 | 100 |
| Sample A Group | 11**/12 | 91.7 |
| Sample A Group | 12**/12 | 100 |
| Sample A Group | 12**/12 | 100 |
| Sample A Group | 12**/12 | 100 |
| Sample A Group | 12**/12 | 100 |
| Sample A Group | 12**/1.2 | 100 |
| Bone Wax Group | 12**/12 | 100 |

** P<0.01 vs control group.

The residues of experimental materials were examined in all defects, no residue was found in Control Group, Sample B Group, Sample C Group, Sample D Group, Sample E Group or Sample F Group, a small amount of the residues were observed in the defects of Sample A Group, while the bone wax completely remained in the defects of Bone wax Group without showing any sign of degradation. The bone healing score was determined for the cranial defects 6 weeks after the surgery, the results revealed that Sample A Group, Sample B Group, Sample C Group, Sample D Group, Sample E Group and Sample F Group all had obviously lower scores than the control (P<0.05), indicating that the 9 samples can promote the healing of cranial bone of the rabbits, while the difference among the 9 samples was not significant; the difference between the Bone wax Group and Control Group was also not significant (P>0.05). See Table 3.

Table3 Residues and bone healing scores in each group of rabbit cranial defects

| Group | Residues amount | Bone healing scores |
|---|---|---|
| Control Group | - | 2.11±0.86 |
| Sample A Group | Small amount | 1.34±0.43* |
| Sample B Group | Not detected | 1.44±0.25* |
| Sample C Group | Not detected | 1.29±0.45* |
| Sample D Group | Not detected | 1.54±0.23* |
| Sample E Group | Not detected | 1.36±0.23* |
| Sample F Group | Not detected | 1.41±0.23* |
| Sample G Group | Not detected | 1.48±0.43* |
| Sample H Group | Not detected | 1.39±0.23* |
| Sample I Group | Not detected | 1.46±0.23* |
| Bone Wax Group | Large amount | 2.06±0.63 |

* P<0.05 vs control group.

## EXPERIMENT 3

Experimental Purpose: the purpose of the experiment was to determine the effects of the medical absorbable hemostatic and wound healing promoting material for bone wounds on healing of long bones in dogs.

Experimental Methods: 10 healthy adult dogs were provided, and a total of 4 circular bone defects were drilled for the long bones of four limbs in each dog (i.e., one bone defect in each limb), hence a total of 40 bone defects were made, during the surgery Sample C Sample E and Sample H of the absorbable hemostatic and wound healing promoting material for bone wounds of the present invention were randomly applied to the defects, which defects were then divided into 5 groups, i.e., Control Group, Sample C Group, Sample E Group, Sample H Group and Bone Wax Group, with 8 defects in each group. The animals were sacrificed 6 weeks after the surgery, specimens of femoral defects were harvested with a rim of surrounding bone at least 1.5 cm from the defect edge, the harvested specimens were then fixed, embedded with paraffin, sliced using conventional methods, examined and photographed using a fluorescence microscopy with ultraviolet light. Tetracycline and calcein can bind to newly formed bone at the bone/osteoid (unmineralized bone) interface where they show linear fluorescence, hence the amount of bone formed during the 6-week interval was calculated by measuring the distance between two fluorescent label lines to determine the mineral apposition rate (MAR), an index of osteoblast activity, i.e., bone formation rate.

$$MAR = \frac{\text{distance between the two fluorescent label lines (μm)}}{\text{Time interval between administration of the two markers}}$$

Experiment Results: the results of mineral apposition rate determined 6 weeks after the surgery revealed that: Sample C Group, Sample E Group and Sample H Group all had significantly higher mineral apposition rates than Control Group as well as the Bone Wax Group ($P<0.05$). See Table 4.

Table 4. Mineral apposition rate in each group

| Group | Mineral apposition rate ($\mu$m/d) |
|---|---|
| Control Group | $2.02\pm0.34$ |
| Sample C Group | $3.88\pm0.84$* # |
| Sample E Group | $3.94\pm0.94$*# |
| Sample H Group | $3.52\pm0.98$*# |
| Bone Wax Group | $1.96\pm0.48$ |

* $P<0.05$ as compared with the control, # $P<0.05$ as compared with Bone Wax Group

**Claims**

1. A medical absorbable hemostatic material for bone wounds, **characterized in that**, it consists of 40-95% of a base material and 5-60% of an adjuvant, based on weight percent;
   wherein the base material is an oligosaccharide, a polysaccharide or a mixture of the oligosaccharide and the polysaccharide;
   and the adjuvant includes: (1) one or more polyhydric alcohols, and

   (2) one or more vegetable oils, and
   (3) one or more emulsifying agents.

2. The medical absorbable hemostatic material for bone wounds as claimed in claim 1, **characterized in that**, the mixture of the oligosaccharide and the polysaccharide contains 10-90% of the oligosaccharide and 10-90% of the polysaccharide, based on weight percent.

3. The medical absorbable hemostatic material for bone wounds as claimed in claim 1 or 2, **characterized in that**, the oligosaccharide is selected from the group consisting of maltose, isomaltose, coupling sugar, soybean sugar, galactose, fructose, sucrose, xylose, maltitol prepared by hydrogenation reactions, soybean sugar alcohol prepared by hydrogenation reactions, galactitol prepared by hydrogenation reactions, fructose alcohol prepared by hydrogenation reactions, sucrose alcohol prepared by hydrogenation reactions and xylitol prepared by hydrogenation reactions; the oligosaccharide has a molecular weight in the range from 160 to 20,000, and the oligosaccharide has a viscous power in the range from 50 to 700 g.mm at room temperature.

4. The medical absorbable hemostatic material for bone wounds as claimed in claim 1 or 2, **characterized in that**, the polysaccharide is selected from the group consisting of carboxymethyl starch, hydroxypropyl starch, pre-gelatinized starch, pharmaceutically acceptable starch, dextrin, alpha-cyclodextrin, beta-cyclodextrin, hydroxypropyl beta-cyclodextrin, carboxymethyl beta-cyclodextrin, sulfobutyl ether-$\beta$-cycloextrin, gamma-cyclodextrin; the polysaccharide has a molecular weight in the range from 10,000 to 1,000,000, has a water absorption rate above 5, and has a viscous power in the range from 10 to 300 g.mm at room temperature.

5. The medical absorbable hemostatic material for bone wounds as claimed in claim 1, **characterized in that**, the adjuvant includes 30-70% of the polyhydric alcohol, 20-60% of the vegetable oil, and 1-10% of the emulsifying agent, based on weight percent;
the polyhydric alcohol comprises at least one selected from medical glycerin and propylene glycol;
the vegetable oil comprises at least one selected from the group consisting of olive oil, soybean oil, hydrogenated soybean oil, hydrogenated castor oil and refined corn oil; and
the emulsifying agent comprises at least one selected from the group consisting of soybean phospholipid, Tween, polysorbate and sucrose stearate.

6. The medical absorbable hemostatic material for bone wounds as claimed in claim 1, **characterized in that**, the absorbable hemostatic material for bone wounds has a bond strength $\geq$ 0.08 mPa, has a bond strength under water immersion $\geq$ 0.03 mPa, and has an amount of water absorption $\geq$ 0.5ml/g.

7. A method of preparing a medical absorbable hemostatic material for bone wounds according to any of claims 1 to 6, comprising mixing a base material and an adjuvant at prescribed amounts by chemical blending and latex blending, cooling to form a solid lump, packaging, and sterilizing, wherein the blendings of the base material and the adjuvant are conducted at a temperature between 40 °C and 80°C.

8. The method of preparing a medical absorbable hemostatic material for bone wounds as claimed in claim 7, **characterized in that**, the chemical blending and the latex blending comprise the steps of:

(1) pretreatment of the base material: washing a polysaccharide with a solvent followed by a drying process to remove impurities and microorganisms from the polysaccharide, and dissolving an oligosaccharide followed by a filtering process to remove impurities and microorganisms from the oligosaccharide;
(2) putting a prescribed amount of the adjuvant into a reaction kettle, then sealing and vacuumizing the reaction kettle, heating the adjuvant to a temperature between 30 °C and 50°C and maintaining the temperature, stirring the adjuvant for 0.5-2 hours;
(3) putting a prescribed amount of the base material into the reaction kettle, then sealing and vacuumizing the reaction kettle, heating the mixture in the reaction kettle to a temperature between 40 °C and 80°C and maintaining the temperature, stirring the mixture for 2-6 hours;
(4) pouring the mixture in the reaction kettle into a mold for molding, immediately placing the mold in an environment having a temperature between 0 °C and 4°C, cooling the mold for 0.5-2 hours to obtain a solid lump product;
(5) packaging the product followed by sterilization through irradiation or oxirane.

9. The method of preparing a medical absorbable hemostatic material for bone wounds as claimed in claim 8, **characterized in that**, the vacuum degree in the reaction kettle is between 10KPa and 30KPa after the vacuum processes.

10. The use of a medical absorbable hemostatic material for bone wounds according to any of claims 1 to 6 in the manufacture of a medicament for the hemostasis of bone wounds and for promoting healing of bone wounds in humans and animals.

**Patentansprüche**

1. Ein resorbierbares medizinisches Blutstillungsmittel für Knochenwunden, **dadurch gekennzeichnet, dass** es aus 40 - 95% eines Grundstoffs und aus 5 - 60% eines Adjuvans besteht, bezogen auf Gewichtsprozent;
wobei der Grundstoff ein Oligosaccharid, ein Polysaccharid oder eine Mischung aus dem Oligosaccharid und dem Polysaccharid ist; und
wobei das Adjuvans beinhaltet:

(1) einen oder mehrere mehrwertige Alkohole, und
(2) ein oder mehrere Pflanzenöle, und
(3) einen oder mehrere Emulgatoren.

**2.** Das resorbierbare medizinische Blutstillungsmittel für Knochenwunden gemäß Anspruch 1, **dadurch gekennzeichnet, dass** die Mischung aus dem Oligosaccharid und dem Polysaccharid 10 - 90% des Oligosaccharids und 10 - 90% des Polysaccharids, bezogen auf Gewichtsprozent, enthält.

**3.** Das resorbierbare medizinische Blutstillungsmittel für Knochenwunden gemäß Anspruch 1 oder 2, **dadurch gekennzeichnet, dass** das Oligosaccharid ausgewählt ist aus der Gruppe bestehend aus Maltose, Isomaltose, Kopplungszucker, Sojabohnen-Zucker, Galaktose, Fruktose, Sucrose, Xylose, Maltit hergestellt durch Hydrierreaktionen, Sojabohnen-Zuckeralkohol hergestellt durch Hydrierreaktionen, Galaktit hergestellt durch Hydrierreaktionen, Fruktose-Alkohol hergestellt durch Hydrierreaktionen, Sucrose-Alkohol hergestellt durch Hydrierreaktionen und Xylit hergestellt durch Hydrierreaktionen; wobei das Oligosaccharid eine Molekularmasse im Bereich von 160 bis 20.000 aufweist und wobei das Oligosaccharid eine Zähigkeitskraft im Bereich von 50 bis 700 g.mm bei Raumtemperatur aufweist.

**4.** Das resorbierbare medizinische Blutstillungsmittel für Knochenwunden gemäß Anspruch 1 oder 2, **dadurch gekennzeichnet, dass** das Polysaccharid ausgewählt ist aus der Gruppe bestehend aus Carboxymethylstärke, Hydroxypropylstärke, vorgelatinierte Stärke, pharmazeutisch akzeptable Stärke, Dextrin, Alpha-Cyclodextrin, Beta-Cyclodextrin, Hydroxypropyl-Beta-Cyclodextrin, Carboxymethyl-Beta-Cyclodextrin, Sulfobutyl-Ether-$\beta$-Cyclodextrin, Gamma-Cyclodextrin; wobei das Polysaccharid ein Molekulargewicht im Bereich von 10.000 bis 1.000.000, eine Wasseraufnahmerate über 5, und eine Zähigkeitskraft im Bereich von 10 bis 300 g.mm bei Raumtemperatur aufweist.

**5.** Das resorbierbare medizinische Blutstillungsmittel für Knochenwunden gemäß Anspruch 1, **dadurch gekennzeichnet, dass** das Adjuvans 30 - 70% des mehrwertigen Alkohols, 20 - 60% des Pflanzenöls, und 1 - 10% des Emulgators, bezogen auf Gewichtsprozent, beinhaltet; wobei der mehrwertige Alkohol zumindest eines ausgewählt aus medizinischem Glycerin und Propylenglykol umfasst; wobei das Pflanzenöl zumindest eines ausgewählt aus der Gruppe bestehend aus Olivenöl, Sojabohnenöl, hydriertes Sojabohnenöl, hydriertes Rizinusöl und raffiniertes Maisöl umfasst; und wobei der Emulgator zumindest eines ausgewählt aus der Gruppe bestehend aus Sojabohnen-Phospholipid, Tween, Polysorbat und Sucrosestearat umfasst.

**6.** Das resorbierbare medizinische Blutstillungsmittel für Knochenwunden gemäß Anspruch 1, **dadurch gekennzeichnet, dass** das resorbierbare Blutstillungsmittel für Knochenwunden eine Bindefestigkeit $\geq$ 0,08 mPa, eine Bindefestigkeit bei Wasserimmersion $\geq$ 0,03 mPa, und einen Betrag der Wasseraufnahme $\geq$ 0,5 ml/g aufweist.

**7.** Verfahren zur Herstellung eines resorbierenden medizinischen Blutstillungsmittels für Knochenwunden gemäß einer der Ansprüche 1 bis 6, umfassend das Mischen eines Grundstoffs und eines Adjuvans mit vorgeschriebenen Mengen durch chemisches Verschneiden und Latex-Verschneiden, Abkühlung, um ein festes Stück zu bilden, Verpacken, und Sterilisierung, wobei die Verschnitte des Grundstoffs und des Adjuvans bei einer Temperatur zwischen 40°C und 80°C durchgeführt werden.

**8.** Das Verfahren zur Herstellung eines resorbierenden medizinischen Blutstillungsmittels für Knochenwunden gemäß Anspruch 7, **dadurch gekennzeichnet, dass** das chemische Verschneiden und das Latex-Verschneiden die Schritte umfassen:

(1) Vorbehandlung des Grundstoffs: Waschen eines Polysaccharids mit einem Lösungsmittel gefolgt von einem Trocknungsprozess, um Unreinheiten und Mikroorganismen von dem Polysaccharid zu entfernen, und Auflösen eines Oligosaccharids gefolgt von einem Filterprozess, um Unreinheiten und Mikroorganismen von dem Oligosaccharid zu entfernen;
(2) Einbringen einer vorgeschrieben Menge des Adjuvans in einen Reaktionskessel, anschließend Verschließen und Evakuieren des Reaktionskessels, Erhitzen des Adjuvans auf eine Temperatur zwischen 30°C und 50°C und Aufrechterhalten der Temperatur, Rühren des Adjuvans für 0,5 - 2 Stunden;
(3) Einbringen einer vorgeschriebenen Menge des Grundstoffs in den Reaktionskessel, anschließend Verschließen und Evakuieren des Reaktionskessels, Erhitzen der Mischung in dem Reaktionskessel auf eine Temperatur zwischen 40 °C und 80 °C und Aufrechterhalten der Temperatur, Rühren der Mischung für 2 - 6 Stunden;
(4) Einfüllen der Mischung in dem Reaktionskessel in eine Form zum Formen, sofortiges Platzieren der Form in einer Umgebung, die eine Temperatur zwischen 0 °C und 4 °C aufweist, Abkühlen der Form für 0,5 - 2 Stunden, um ein festes Stückerzeugnis zu erhalten;
(5) Verpacken des Erzeugnisses, gefolgt von einer Sterilisation durch Bestrahlung oder Oxiran.

**9.** Das Verfahren zur Herstellung eines resorbierenden medizinischen Blutstillungsmittels für Knochenwunden gemäß Anspruch 8, **dadurch gekennzeichnet, dass** der Vakuumwert in dem Reaktionskessel zwischen 10KPa und 30KPa nach den Vakuumierungsprozessen liegt.

**10.** Die Verwendung eines resorbierbaren medizinischen Blutstillungsmittels für Knochenwunden gemäß einer der Ansprüche 1 bis 6 bei der Herstellung eines Medikaments zur Blutstillung von Knochenwunden und zur Förderung der Heilung von Knochenwunden bei Menschen und Tieren.

**Revendications**

**1.** Matériau médical hémostatique absorbable pour des lésions osseuses, **caractérisé en ce qu'**il est constitué de 40 à 95 % d'un matériau de base et de 5 à 60 % d'un adjuvant, sur la base des pourcentages en poids ;
dans lequel le matériau de base est un oligosaccharide, un polysaccharide ou un mélange de l'oligosaccharide et du polysaccharide ;
et l'adjuvant contient :

(1) un ou plusieurs alcools polyvalents, et
(2) une ou plusieurs huiles végétales, et
(3) un ou plusieurs agents émulsionnants.

**2.** Matériau médical hémostatique absorbable pour des lésions osseuses selon la revendication 1, **caractérisé en ce que** le mélange de l'oligosaccharide et du polysaccharide contient 10 à 90% de l'oligosaccharide et 10 à 90 % du polysaccharide, sur la base des pourcentages étant en poids.

**3.** Matériau médical hémostatique absorbable pour des lésions osseuses selon la revendication 1 ou la revendication 2, **caractérisé en ce que** l'oligosaccharide est choisi dans le groupe constitué par le maltose, l'isomaltose, le Coupling Sugar (glycosylsaccharose), le sucre de soja, le galactose, le fructose, le saccharose, le xylose, le maltitol préparé par des réactions d'hydrogénation, l'alcool de sucre de soja préparé par des réactions d'hydrogénation, le galactitol préparé par des réactions d'hydrogénation, l'alcool de fructose préparé par des réactions d'hydrogénation, l'alcool de saccharose préparé par des réactions d'hydrogénation et le xylitol préparé par des réactions d'hydrogénation ; l'oligosaccharide a une masse moléculaire située dans la plage allant de 160 à 20 000, et l'oligosaccharide a un pouvoir de viscosité situé dans la plage allant de 50 à 700 g.mm à la température ambiante.

**4.** Matériau médical hémostatique absorbable pour des lésions osseuses selon la revendication 1 ou la revendication 2, **caractérisé en ce que** le polysaccharide est choisi dans le groupe constitué par le carboxyméthyl-amidon, l'hydroxypropyl-amidon, l'amidon pré-gélatinisé, l'amidon pharmaceutiquement acceptable, la dextrine, l'$\alpha$-cyclo-dextrine, la $\beta$-cyclodextrine, l'hydroxypropyl-$\beta$-cyclodextrine, la carboxyméthyl-$\beta$-cyclodextrine, la sulfobutyléther-$\beta$-cyclodextrine, la -cyclodextrine ; et le polysaccharide a une masse moléculaire située dans la plage allant de 10 000 à 1 000 000, a un taux d'absorption d'eau supérieur à 5, et a un pouvoir de viscosité situé dans la plage allant de 10 à 300 g.mm à la température ambiante.

**5.** Matériau médical hémostatique absorbable pour des lésions osseuses selon la revendication 1, **caractérisé en ce que** l'adjuvant contient 30 à 70 % de l'alcool polyvalent, 20 à 60 % de l'huile végétale, et 1 à 10 % de l'agent émulsionnant, sur la base des pourcentages en poids ;
l'alcool polyvalent comprend au moins un alcool choisi parmi le glycérol à usage médical et le propylèneglycol ;
l'huile végétale comprend au moins une huile choisie dans le groupe constitué par l'huile d'olive, l'huile de soja, l'huile de soja hydrogénée, l'huile de ricin hydrogénée et l'huile de maïs raffinée ; et
l'agent émulsionnant comprend au moins un agent choisi dans le groupe constitué par les phospholipides de soja, Tween, les polysorbates et le stéarate de saccharose.

**6.** Matériau médical hémostatique absorbable pour des lésions osseuses selon la revendication 1, **caractérisé en ce que** le matériau médical hémostatique absorbable pour des lésions osseuses a une force de collage $\geq$ 0,08 mPa, a une force de collage sous immersion dans l'eau $\geq$ 0,03 mPa, et a une quantité d'absorption d'eau $\geq$ 0,5 ml/g.

**7.** Procédé pour préparer un matériau médical hémostatique absorbable pour des lésions osseuses selon l'une quelconque des revendications 1 à 6, comprenant le mélange d'un matériau de base et d'un adjuvant en des quantités prescrites par mélange chimique et mélange de latex, le refroidissement pour former une masse solide, le conditionnement, et la stérilisation, dans lequel les mélanges du matériau de base et de l'adjuvant sont effectués à une température comprise entre 40 °C et 80 °C.

8. Procédé pour préparer un matériau médical hémostatique absorbable pour des lésions osseuses selon la revendication 7, **caractérisé en ce que** le mélange chimique et le mélange de latex comprennent les étapes de :

(1) prétraitement du matériau de base, lavage d'un polysaccharide avec un solvant suivi d'un séchage pour éliminer les impuretés et les microorganismes du polysaccharide, et dissolution d'un oligosaccharide suivie d'une filtration pour éliminer les impuretés et les microorganismes de l'oligosaccharide ;
(2) introduction d'une quantité prescrite de l'adjuvant dans une marmite réactionnelle, puis scellage et mise sous vide de la marmite réactionnelle, chauffage de l'adjuvant à une température comprise entre 30 °C et 50 °C et maintien de la température, agitation de l'adjuvant pendant 0,5 à 2 heures ;
(3) introduction d'une quantité prescrite du matériau de base dans la marmite réactionnelle, puis scellage et mise sous vide de la marmite réactionnelle, chauffage du mélange dans la marmite réactionnelle à une température comprise entre 40 °C et 80 °C et maintien de la température, agitation du mélange pendant 2 à 6 heures ;
(4) versage du mélange de la marmite réactionnelle dans un moule pour moulage, introduction immédiate du moule dans un environnement ayant une température comprise entre 0 °C et 4 °C, et refroidissement du moule pendant 0,5 à 2 heures pour obtenir un produit en masse solide ;
(5) conditionnement du produit suivi d'une stérilisation par irradiation ou avec de l'oxirane.

9. Procédé pour préparer un matériau médical hémostatique absorbable pour des lésions osseuses selon la revendication 8, **caractérisé en ce que** le degré de vide dans la marmite réactionnelle est compris entre 10 kPa et 30 kPa après le traitement sous vide.

10. Utilisation d'un matériau médical hémostatique absorbable pour des lésions osseuses selon l'une quelconque des revendications 1 à 6 dans la fabrication d'un médicament destiné à l'hémostase de lésions osseuses et à favoriser la cicatrisation de lésions osseuses chez les humains et les animaux.

**EP 2 626 092 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 00110393
- CN 200610093091
- CN 200510035251
- CN 3229540
- US 5143730 A
- US 4439420 A
- WO 2005034816 A1
- US 200506214 A1